# EUROPEAN PATENT SPECIFICATION

(11) **EP 2 028 162 B1**
(45) Date of publication and mention of the grant of the patent: **16.03.2011**
(21) Application number: 07425508.4
(22) Date of filing: 03.08.2007
(51) Int. Cl.: C02F 11/04, C02F 3/28

(54) **Reactor for the anaerobic production of biogas from pre-treated wet waste and stirring method in such reactor**
Reaktor für die anaerobe Produktion von Biogas aus vorbehandeltem feuchtem Abfall und Rührverfahren in solchem Reaktor
Reacteur pour la production anaérobique de biogaz d'ordures humides prétraités et procédé d' agitation dans cette reacteur

(43) Date of publication of application: 25.02.2009
(73) Proprietor: ACEA Pinerolese Industriale S.p.A., 10064 Pinerolo (IT)
(72) Inventor: Bellintani Marco, 10060 Pinasca (IT)
(74) Representative: Bergadano, Mirko

(56) References cited:
- WO-A-89/00151
- WO-A-98/24730
- WO-A-02/100784
- DE-C1- 19 800 224
- US-A- 5 651 890
- US-B1- 6 254 775
- NAH I W ET AL: "Mechanical pretreatment of waste activated sludge for anaerobic digestion process" WATER RESEARCH, ELSEVIER, AMSTERDAM, NL, vol. 34, no. 8, June 2000 (2000-06), pages 2362-2368, XP004193854 ISSN: 0043-1354

## Description

The present invention relates to a reactor for the anaerobic production of biogas from pre-treated wet waste and to a stirring method of a mixture comprising such pre-treated wet waste in such reactor.

There are known biogas production plants comprising a feeding station of wet waste in bags, a pre-treatment unit of the aforesaid wet waste, and a reactor in which the pre-treated wet waste bio-chemically reacts with appropriate micro-organisms. Such biochemical reaction generates biogas useable as source of energy and a reject, known as digestate, meant to be conveyed to a composting plant.

More specifically, the pre-treatment unit sequentially performs on the wet waste a first crushing adapted to break the bags, a sieving adapted to eliminate predetermined impurities, and a second crushing contemplated to take the wet waste itself to a sufficiently small dimension to be made suitable for the subsequent biochemical reaction.

Bio-digestion plants further comprise a tank interposed between the pre-treatment unit and the reactor along the wet waste feed line.

More specifically, such tank is adapted to dilute in water and heat up the wet waste coming from the pre-treatment unit, concurrently separating a fraction of non-biodegradable discards from the wet waste itself.

The reactor essentially comprises a first chamber receiving the water and wet waste mixture from the tank, in which the inert materials are eliminated by precipitation; and a second chamber, in which the aforesaid mixture is made to react, under anaerobic conditions, with micro-organisms generating the biogas and the digestate.

More specifically, the second chamber surrounds the first chamber and receives the aforesaid mixture, by overflowing, from the first chamber itself.

The biogas predominantly consists of methane and for the remaining part of carbon dioxide and other components, and is accumulated in a storage gasometer, from where it is subsequently conveyed to a use station, e.g. a co-generation plant.

A first fraction of the digestate is accumulated, by gravity, on a bottom portion of the second chamber, from where it is subsequently conveyed to a composting plant, after having been dehydrated.

A second fraction of the digestate, lighter than the aforesaid first fraction, floats on the free surface of the wet waste mixture which is reacting with the micro-organisms in the second chamber.

Furthermore, the reactors of the known type comprise a vertical shaft accommodated within the second chamber, provided with a plurality of radial appendixes and rotational about an axis thereof so as to maintain the water and pre-treated wet waste mixture arriving from the first chamber constantly in motion during the reaction with the micro-organisms.

Such state of motion is needed to ensure a high efficacy of the biochemical reaction between the micro-organisms and the wet waste and water mixture present in the second chamber, and to avoid the partial solidification of the mixture itself.

It is felt in the sector the need to increase the efficiency of the transformation and to prevent the solidification of the mixture in the zones of the second chamber most distant from the shaft without interfering with the correct operation of the shaft itself.

It is the object of the present invention to make a reactor for the anaerobic production of biogas from pre-treated wet waste, which allows to simply and cost-effectively fulfill the aforesaid need.

The aforesaid object is reached by the present invention in that it relates to a reactor for the anaerobic production of biogas from pre-treated wet waste as defined in claim 1.

For a better understanding of the present invention, a preferred embodiment will be described below, only by way of non-limitative example and with reference to the accompanying figures, in which:
- figure 1 is a functional diagram of an anaerobic digestion plant of pre-treated wet waste comprising a reactor according to the present invention with parts removed for clarity;
- figure 2 shows an axial section of the reactor of the plant in figure 1;
- figure 3 is a top view of the reactor in figure 2; and

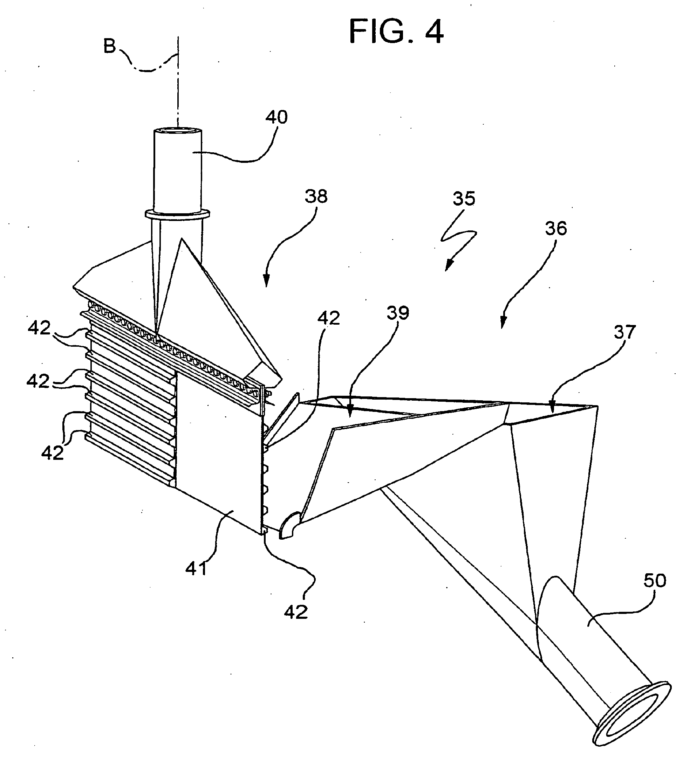

- figure 4 shows a highly enlarged perspective view of some details of the reactor in figures 2 and 3.

With reference to the accompanying figures, numeral 1 indicates a pre-treated wet waste anaerobic bio-digestion plant.

In greater detail, the plant 1 essentially comprises:
- a pre-treatment unit 2 (only diagrammatically shown) receiving pre-treated wet waste in bags;
- a tank 3 adapted to dilute the pre-treated wet waste in water and heat it to approximately 65 degrees centigrade; and
- a reactor 4 receiving a wet waste and water mixture exiting from the tank 3, and in which such mixture reacts with appropriate micro-organisms generating biogas and a reject, known as digestate.

Specifically, the reactor 4 discontinuously processes fair volumes of the aforesaid mixture, i.e. receives in input a volume of mixture, and discharges such volume, once the mixture has completed the reaction with the micro-organisms.

A heavy fraction of the digestate precipitates, by gravity, inside the reactor 4, while a light fraction of the digestate itself floats on a free surface 61 of the reacting wet waste and water mixture.

Such light fraction of the digestate essentially consists of organic component particles.

Inside the unit 2, the wet waste undergoes a pre-treatment essentially consisting of a first, course crushing aimed at tearing the bags, of a sieving adapted to deprive the wet waste from predetermined impurities and of a second crushing adapted to make the wet waste of sufficiently small dimensions to be able to react with the micro-organisms in the reactor.

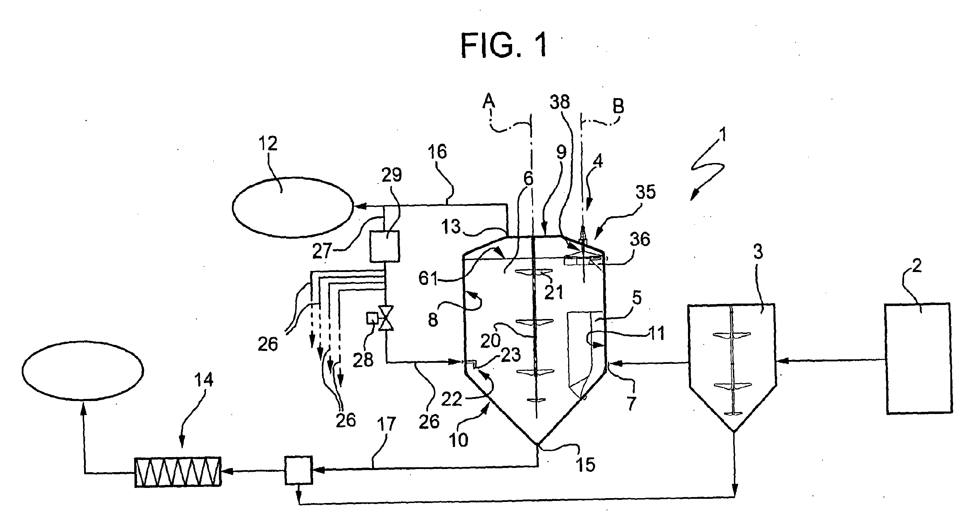

The tank 3 is adapted to maintain the wet waste thus pre-treated in a state of stirring (in a manner only diagrammatically shown in figure 1) so as to separate a faction consisting of non-biodegradable discards from the wet waste and water mixture.

Such wet waste and water mixture, after having left the tank 3, is pumped (in a manner not shown) into the reactor 4.

The reactor 4 (figures 1, 2 and 3) essentially comprises:
- a pre-chamber 5 receiving the waste and water mixture from the tank 3 by means of an inlet mouth 7 and adapted to separate, by precipitation, a fraction of inert materials from the aforesaid mixture; and
- a reaction chamber 6 surrounding the pre-chamber 5, receiving the waste and water mixture from the pre-chamber 5 itself, and in which such mixture biochemically reacts under anaerobic conditions with the micro-organisms generating biogas and digestate.

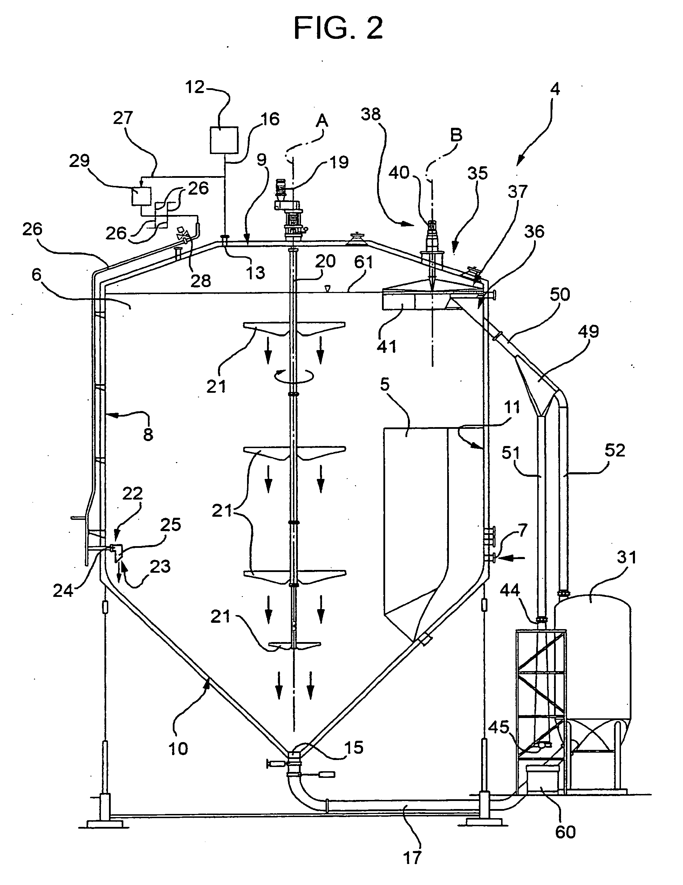

In greater detail (figure 2), the reactor 4 extends along an axis A vertically arranged in use and is delimited by a main cylindrical wall 8 of axis A, by a top wall 9 and by a truncated cone bottom wall 10 having a decreasing radial dimension proceeding from the wall 8 opposing the wall 9.

The walls 9, 10 are arranged on opposite sides of the wall 8.

The wall 8 laterally delimits the chamber 6 with respect to axis A, and faces away from axis A, while the walls 9, 10 are incident and transversal to axis A.

The pre-chamber 5 protrudes overhanging in the reactor 4 from a segment 11 of the wall 8 and from a segment, contiguous to the segment 11, of the wall 10.

Furthermore, the pre-chamber 5 extends in the reactor 4 in an eccentric position with respect to axis A and on one side of axis A itself.

The pre-chamber 5 essentially comprises a closed bottom meant to collect the inert materials separated by precipitation, and a side wall extending from the segment 11 of the wall 8.

The segment 11 of the wall 8 is crossed by the inlet mouth 7, which is fluidically connected to the tank 3 to allow the water and wet waste mixture to reach the pre-chamber 5 itself.

Finally, the pre-chamber 5 is open in the upper part and on the side opposite to the closed bottom to allow the wet waste and water mixture to overflow into the chamber 6.

The chamber 6 occupies the volume of the reactor 4 not occupied by the pre-chamber 5, is selectively connectable to a biogas storage gasometer 12 (only diagrammatically indicated in figures 1 and 2) by means of an outlet mouth 13 obtained in the wall 9, and is selectively connectable to a treatment plant 14 (only diagrammatically indicated in figure 1) of the heavy fraction of the digestate, by means of an outlet mouth 15 obtained at an end of the wall 10, opposite to the wall 8.

More specifically, a conveying pipe 16 of the biogas is interposed between the outlet mouth 13 and the gasometer 12, and a conveying pipe 17 of the heavy digestate fraction is interposed between the outlet mouth 15 and the plant 14.

The biochemical reaction occurs at a temperature of approximately 50 degrees centigrade and the wet waste and water mixture remains in the chamber 6 itself for at least fourteen days.

Furthermore, the reactor 4 comprises a stirring system interacting with the wet waste and water mixture to maintain the mixture itself in a state of constant motion preventing the partial solidification thereof, and in order to promote the progress of the reaction with the micro-organisms.

The stirring system comprises a shaft 20 accommodated in the chamber 6, turnable about axis A, and provided with a plurality of disc-shaped appendixes 21, four in the case in point, radially protruding from the shaft 20 itself and interacting with the wet waste and water mixture.

The shaft 20 is actuated by a motor 19 (shown only in figure 2), extends overhanging from the wall 9 and presents a free end opposite to the wall 9 itself, accommodated in the chamber 6 and surrounded by the wall 10.

Advantageously, the stirring system comprises supplying means of a plurality of biogas jets in the chamber 6; the supplying means are feedable with the biogas produced by the biochemical reaction in progress in the chamber 6, pass through the wall 8, and are provided with a plurality of supplying mouths 23, five in the case in point (only one of which is shown in figure 2), of corresponding biogas jets in the chamber 6; the mouths 23 are arranged inside the chamber 6 in corresponding positions adjacent to the wall 8 to maintain corresponding regions of the mixture spaced from the shaft 20 and adjacent to the wall 8 itself in a continuous state of stirring.

In greater detail, the supplying means comprise:
- a plurality of pipes 26, five in the case in point, connected to the pipe 16 and each presenting a main portion extending externally to the chamber 6 and an end portion crossing the wall 8; and
- a plurality of nozzles 22 (only one of which is shown in figure 2) provided with corresponding mouths 23 and each fluidically connected to a corresponding pipe 26 and adapted to supply the corresponding biogas jets inside the chamber 6.

In greater detail, each pipe 26 is fluidically interposed between a corresponding deviation 27 of the pipe 16 and the corresponding nozzle 22.

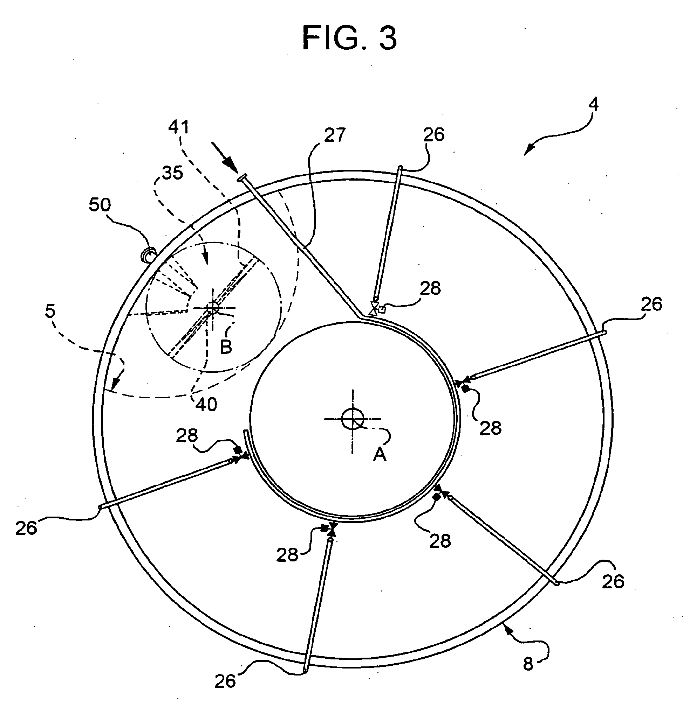

Along such deviation 27, there is interposed a compressor 29 (only diagrammatically shown in figures 2 and 3) adapted to increase the pressure of the biogas fed to pipes 26 and supplied into chamber 6.

The pipes 26 are secured to the external surfaces of the walls 8 and 9.

Along the pipes 26 there are interposed corresponding valves 28 (only one of which is shown in figures 1 and 2) electronically controlled by a control unit (not shown in the accompanying figures).

More specifically, the valves 28 are controlled so that only one of them is opened, while the other valves 28 remain closed.

The opening sequence of the valves 28 depends on the viscosity of the water and wet waste mixture and is such to promote a displacement action of the aforesaid mixture towards the wall 9.

By controlling the valves 28 so that the biogas flows only along one pipe 26 at a time, and thus so that only one mouth 23 supplies biogas into chamber 6 at a time, the formation of a circular crown of solidified wet waste on the internal surface of the chamber 6 is also prevented.

Each nozzle 22 is arranged inside the chamber 6 and comprises a first end receiving the biogas from the corresponding pipe 26, and a second free end, opposite to the first end, defining the outlet mouth 23 of the corresponding biogas jet.

Furthermore, each nozzle 22 comprises a first segment 24 extending in a radial direction with respect to axis A and provided with the corresponding first end, and a second segment 25 extending from the segment 24 parallelly to axis A and towards the outlet mouth 15. The segment 25, moreover, defines the mouth 23.

The biogas jets exiting from the mouths 23 are thus directed parallelly to axis A, towards the wall 10 and in a position adjacent to the wall 8. In such manner, such jets maintain the wet waste and water mixture in movement in corresponding regions of the chamber 6 adjacent to the wall 8 and away from the shaft 20.

Specifically, each mouth 23 lays on an inclined plane with respect to axis A and is shaped so as to move closer to the wall 9, proceeding from the corresponding pipe 26 towards the shaft 20.

Such shape of the mouths 23 slows down the speed of the biogas passing through the mouths 23 themselves and contrasts the occlusion of the mouths 23 by the reacting water and wet waste mixture.

The reactor 4 comprises a tank 31 fluidically connected to the chamber 6 and crossed by the pipe 17. The tank 31, being fluidically connected to the chamber 6, is normally full of biogas.

Furthermore, the reactor 4 comprises a removal device 35 adapted to carry away from the chamber 6 some of the light fraction of the digestate floating on the free surface 61.

The device 35 is arranged in an eccentric position with respect to axis A and higher than the appendix 21 arranged in the highest position and, thus, closest to the wall 9. Furthermore, the device 35 is essentially arranged at the same distance as the pre-chamber 5 from the axis A.

The device 35 essentially comprises:
- a collection element 36 fixed to the reactor 4, and defining a basin 37 open towards the wall 9 and fluidically connected to a discharge tank 60 (shown in figure 2) of the light fraction of the digestate; and
- a conveying element 38 rotational about an axis B, parallel to axis A, to carry away such light fraction and guide it into the basin 37.

More precisely, the element 36 comprises a wall defining a chute 39 adjacent to the element 38, inclined with respect to axis A and immersed in the wet waste and water mixture immediately underneath the free surface 61.

The chute 39 presents a first end edge contiguous to the basin 37 and a second free end edge, opposite to the first end.

Furthermore, the first edge of the chute 39 extends for a shorter length than the second edge of the chute 39 itself.

The chute 39 thus defines a feed surface for the light fraction of the digestate conveyed from the converging element 38 towards the basin 37.

Specifically, the first edge of the chute 39 is arranged in a higher position than the second edge of the chute 39 itself so as to promote the return of the water dragged by the element 38 along with the light fraction of the reject back into the chamber 6.

The basin 37 further presents a pipe 50 fluidically connected to the discharge tank 60 on the opposite side of the wall 9.

The element 38 is rotational about an axis B, parallel to axis A, to carry away such light fraction and guide it along the chute 39 and into the basin 37.

The element 38 specifically comprises a motor (not shown), a shaft 40 operatively connected to the motor itself to be rotationally fed about axis B, and a crossbar 41 integral with the shaft 40 and provided, on both sides, with a plurality of strips 42 of elastically deformable material, specifically rubber, immersed (figures 1 and 2) immediately underneath the free surface 61 and interacting with the light floating fraction to guide it along the chute 39 and towards the tank 37.

The crossbar 41 lays on a plane parallel to axis B and vertically arranged in use, while the strips 42 are orthogonal elongated to axis B.

The crossbar 41 is delimited in the lower part by an edge opposite the shaft 40 and arranged at the same height as the second edge of the chute 39.

The pipe 50 presents a first end connected to the basin 37 and a second end opposite to the first end defined by a branch 49. A tube 52 ending in the tank 31 and a tube 51 ending in the discharge tank 60 of the light fraction of the digestate originate at such branch 49.

Furthermore, the tube 51 presents a pair of reciprocally spaced valves 44, 45 adapted to allow to discharge the light fraction of the digestate without generating leakages of biogas.

More specifically, the valve 44 is interposed between the valve 45 and the branch 49.

The tube 51 is normally full of biogas and of the light fraction of the digestate. Specifically, the light fraction of the digestate is heavier than the biogas and is arranged, by gravity, at the end adjacent to the valve 44 of the portion of the tube 51 interposed between the branch 49 and the valve 44.

The valve 44 is, moreover, displaceable between an opened position in which it allows the passage of the light fraction of the digestate into the portion of the tube 51 interposed between the valves 44, 45, and a second closed position in which it prevents the biogas contained in the tube 51 from reaching the portion of the tube 51 interposed between the valves 44, 45.

The valve 45 is arranged in a closed position when the valve 44 is arranged in the opened position so as to trap the light fraction of the digestate in the portion of the tube 51 comprised between the valves 44, 45.

Otherwise, the valve 45 is arranged in the opened position, when the valve 44 is arranged in the closed position to allow the light fraction of the digestate previously trapped between the valves 44, 45 to reach the discharge tank 60.

In use, the unit 2 performs a plurality of pre-treatments on the wet waste fed in bags. More specifically, such pre-treatments comprise a first crushing aimed at tearing the bags, a sieving adapted to deprive the wet waste of predetermined impurities, and a second crushing contemplated to make the wet waste of sufficiently small dimensions to be able to react with the micro-organisms in the reactor 4.

The wet waste exiting from the unit 2 reach the tank 3, where they are diluted and heated up to approximately sixty-five degrees centigrade and, later, they advance towards the inlet 7 of the pre-chamber 5.

Inside the pre-chamber 5, the inert materials are separated from the mixture and precipitates onto the closed bottom of the pre-chamber 5 itself.

The wet waste and water mixture overflows from the pre-chamber 5 into the chamber 6, where it reacts, at a temperature of approximately 50-55 degrees centigrade and for a time of at least fourteen days, with the micro-organisms present in the chamber 6 itself generating biogas and digestate.

The biogas moves up towards the wall 9, while the heavy fraction of the digestate precipitates by gravity inside the chamber 6 and accumulates on the wall 10.

Otherwise, the light fraction of the biogas floats on the free surface of the wet waste and water mixture.

After the reaction is completed, the outlet mouth 13 is opened and the biogas reaches the gasometer 12 by means of the pipe 16. The biogas is stored in the gasometer 12 and subsequently made available to a co-generation plant.

By means of the appendixes 21, the rotation of the shaft 20 maintains the region of the wet waste and water mixture adjacent to axis A in a constant state of stirring.

Furthermore, part of the biogas which flows along the pipe 16 is deviated along the deviation 27, compressed by the compressor 29 and made available to the pipes 26.

Each pipe 26 supplies the pressurized biogas to the corresponding nozzle 22, which expels a corresponding biogas jet by means of the corresponding mouth 23 in proximity of a corresponding segment of the wall 8 and in the direction of the wall 10.

Such biogas jets contribute to maintain the wet waste and water mixture which is reacting in the chamber 6 at corresponding regions adjacent to the wall 8 and away from the shaft 20 in a state of continuous motion.

Furthermore, the valves 28 are controlled by the control unit so that the pressurized biogas can flow along one only pipe 26 and be introduced into the chamber 6 by means of only one nozzle 22.

The nozzle 22 through which the biogas is supplied into the chamber 6 is selected so as to promote the displacement of the reacting wet waste and water mixture towards the device 35, and to contrast the formation of a circular crown of solidified mixture on the internal surface of the reactor 4.

Specifically, by alternating the nozzle 22 from which the biogas jet exits, such circular crown is broken as soon as it is formed.

When it is necessary to remove the light fraction from the free surface 61, the shaft 40 turns about axis B so that the crossbar 41 pushes the light fraction of the digestate along the chute 39 inside the basin 37. The light fraction is later conveyed to the discharge tank 60 by means of the pipe 50 and the tube 51.

When the light fraction of the digestate is discharged, the valves 44, 45 are both closed so that the light fraction may occupy the segment adjacent to the valve 44 of the portion of the tube 51 interposed between the branch 49 and the valve 44.

More specifically, the light fraction of the digestate, arranging adjacent to the valve 44, pushes the biogas into the segment adjacent to the branch 49 of the portion of the tube 51 interposed between the branch 49 and the valve 44.

Afterwards, the valve 44 is opened so at to allow the light fraction of the digestate to occupy the segment of the tube 51 interposed between the valves 44, 45.

The opening times of the valve 44 are such that most of the biogas remains in the portion of the tube 51 comprised between the branch 49 and the valve 44.

Finally, the valve 44 is closed and the valve 45 is opened allowing to supply the light fraction of the digestate into the discharge tank, in this manner containing the risk of leakages of biogas into the atmosphere.

At the end of the biochemical reaction, the heavy fraction of the digestate is discharged by means of the outlet mouth 15, passes through the pipe 17 and fills the tank 31.

The discharge operation of the heavy fraction of the digestate determines an overpressure in the pipe 17 and in the tank 31, thus elevating the pressure of the biogas contained in the tank 31 itself.

In virtue of the fact that the tank 31 is fluidically connected, by means of the tube 52 and the pipe 50, to the chamber 6, such overpressure is balanced returning the biogas back into the chamber 6 itself.

From an examination of the features of the reactor 4 and of the method according to the invention, the advantages that it allows to obtain are apparent.

Specifically, the gas jets supplied by the nozzles 22 allow to maintain corresponding regions of the chamber 6 adjacent to the wall 8 and spaced from the shaft 20 in constant state of stirring without interfering with the rotation of the shaft 20 itself.

Indeed, the pipes 26 extend outside the reactor 4 and the nozzles 22 are arranged adjacent to the wall 8 and away from the shaft 20.

Furthermore, the nozzles 22 are sufficiently stiff to not be dragged away by the viscous action of the wet waste and water mixture rotating inside the chamber 6.

It is thus avoided the danger that such nozzles 22, once they are dragged away and detached from the corresponding pipes 16, may be dragged away by the viscosity of the waste and water mixture against the shaft 20, preventing the operation thereof and/or causing damage thereto.

Furthermore, in virtue of the fact that the nozzles 22 supply the biogas into the chamber 6 one by one, the displacement of the reacting wet waste and water mixture towards the device 35 is promoted, and the formation of a circular crown of solidified mixture on the internal surface of the reactor 4 is avoided.

It is finally apparent that changes and variants can be made to the reactor 4 and to the method previously described without departing from the scope of protection of the present invention.

Specifically, the reactor 4 could comprise a single nozzle 22 and a single pipe 26.

Furthermore, the end portion of the pipes 26 could be outside the reactor 4 and the nozzle 22 could cross the wall 8 of the reactor 4 itself.

## Claims

1. A reactor (4) for the anaerobic production of biogas from pre-treated wet waste, comprising:
- a reaction chamber (6) feedable with a mixture comprising said wet waste, and adapted to house a biochemical reaction in anaerobic environment, in which said mixture is transformed into biogas and a reject; and
- stirring means (20, 22) comprising at least one shaft (20) rotational inside said chamber (6) about an axis (A) and interacting, in use, with said mixture to maintain it in a state of continuous motion in said chamber (6);
said stirring means (20, 22, 23, 26, 27) comprising supplying means of one biogas jet into said chamber (6); said supplying means (22, 23, 26, 27) being feedable with said biogas produced by said biochemical reaction and passing through a side wall (8) of said chamber (6) facing said shaft (20);
**characterized in that** said supplying means (22, 23, 26, 27) comprise:
- two supplying mouths (23) of said biogas jet inside said chamber (6) in a position adjacent to said side wall (8) to maintain at least one region of said mixture spaced from said shaft (20) and adjacent to said side wall (8) in a continuous state of stirring;
- two nozzles (22) defining supplying mouths (23) of said biogas jet;
- two conveying pipes (26) of said biogas produced in said chamber (6) and fluidically connected to said nozzles (22);
- two adjustment valves (28) each interposed along a corresponding pipe (26); and
- a control unit acting, in use, on said valves (28) so as to maintain at least one of said valves (28) open at a time while the other said valve (28) remains closed, and to allow said biogas to flow along only one pipe (26) at a time and to allow only one corresponding nozzle (22) at a time to supply said biogas jet into said chamber (6); the pipe (26) along which said flow is allowed varying in time, in use, so as to prevent the solidification of said mixture.

2. A reactor according to claim 1, **characterized in that** each said nozzle (22) has a first reception segment (24) of said biogas and a second segment (25) transversally extending with respect to the first segment (24) and defining said supplying mouth (23) of said jet.

3. A reactor according to claim 2, **characterized in that** said mouth (23) lays on an inclined plane with respect to the direction of extension of said second segment (25) of said nozzle (22).

4. A reactor according to claim 2 or 3, **characterized in that** said second segment (25) of said nozzle (22) extends parallelly to said axis (A).

5. A reactor according to any claim from 2 to 4, **characterized in that** said first segment (24) of said nozzle (22) extends orthogonally to said axis (A).

6. A reactor according anyone of preceding claims, **characterized in that** said conveying pipe (26) comprises a main portion extending outside said chamber (6) and presents an end portion crossing said side wall (8) and fluidically connected to said first segment (24) of said nozzle (22).

7. A reactor according to any preceding claim, **characterized in that** said side wall (8) extends about the axis (A), and **in that** said chamber (6) comprises:
- a top wall (9) transverse and incident to said axis (A), arranged on one side of said side wall (8), and defining a first outlet mouth (13) for said biogas generated by said reaction; said shaft (20) protruding overhanging from said top wall (9) inside said chamber (6); and
- a bottom wall (10) transverse and incident to said axis (A), arranged on opposite side of said side wall (8) with respect to said top wall (9), and adapted to collect, by gravity, at least one fraction of said reject; said bottom wall (10) further defining a second outlet mouth (15) for said fraction of said reject.

8. A reactor according to claim 7, when dependent on any claim from 2 to 6, **characterized in that** said second segment (25) of said nozzle (22) extends from said first segment (24) towards said bottom wall (10).

9. A reactor according to any preceding claims, **characterized in that** it comprises a compressor member (29) receiving said biogas produced in said chamber (6), adapted to compress said biogas and fluidically connected to said conveying pipe (26) so as to pressurize said biogas meant to be supplied into said chamber (6).

10. A plant for the production of biogas from wet waste, comprising:
- a pre-treatment unit (2) receiving said wet waste and adapted to crush and sieve said wet waste;
- a reactor (4) according to any preceding claim fluidically connected to said pre-treatment unit (2) and fluidically connected to a treatment unit (14) of said reject; and
- a tank (12) fluidically connected to said reactor (4) and adapted to store said biogas produced in said chamber (6).

## Patentansprüche

1. Reaktor (4) für die anaerobe Produktion von Biogas aus vorbehandeltem nassen Abfall, welcher Folgendes aufweist:
eine Reaktionskammer (6), die mit einer Mischung beschickbar ist, welche aus nassem Abfall besteht, und die geeignet ist, dass in ihr eine biochemische Reaktion in einer anaeroben Umgebung stattfindet, in welcher die Mischung in Biogas und einen Rest umgewandelt wird; und
Rührmittel (20, 22), welche mindestens eine Welle (20) aufweisen, die innerhalb der Kammer (6) um eine Achse (A) drehbar sind, und die im Betrieb mit der Mischung in Wechselwirkung treten, um sie in einem Zustand der kontinuierlichen Bewegung in der Kammer (6) zu halten;
wobei die Rührmittel (20, 22, 23, 26, 27) Liefer- bzw. Einspeismittel für einen Biogasstrahl in die Kammer (6) aufweisen; wobei die Einspeismittel (22, 23, 26, 27) mit Biogas, welches durch die biochemische Reaktion produziert wird, beschickbar sind, und welche durch eine Seitenwand (8) der Kammer (6), welche der Welle (20) zugewandt ist, verlaufen;
**dadurch gekennzeichnet, dass** die Einspeismittel (22, 23, 26, 27) Folgendes aufweisen:
- zwei Einspeisöffnungen (23) für den Biogasstrahl innerhalb der Kammer (6) in einer Position benachbart zu der Seitenwand (8), um zumindest einen Bereich der Mischung, der sich von der Welle (20) beabstandet und benachbart zur Seitenwand (8) befindet, in einem kontinuierlichen Rührstadium zu halten;
- zwei Düsen (22), welche die Einspeisöffnungen (23) des Biogasstrahls definieren;
- zwei Förderleitungen (26) für das Biogas, welches in der Kammer (6) produziert wird, die strömungsmittelmäßig mit den Düsen (22) verbunden sind;
- zwei Einstellventile (28), die jedes entlang der entsprechenden Leitung (26) eingefügt sind; und
- eine Steuereinheit, die im Betrieb auf die Ventile (28) wirkt, um zumindest eines der Ventile (28) zu einem Zeitpunkt offen zu halten, während das andere Ventil (28) geschlossen bleibt, und um zu gestatten, dass Biogas entlang nur einer Leitung (26) zu einem Zeitpunkt fließt, und zu gestatten, dass nur eine entsprechende Düse (22) zu einem Zeitpunkt einen Biogasstrahl in die Kammer (6) liefert; wobei die Leitung (26), entlang welcher die Strömung laufen kann, im Betrieb zeitlich variiert bzw. wechselt, um eine Verfestigung der Mischung zu verhindern.

2. Reaktor gemäß Anspruch 1, **dadurch gekennzeichnet, dass** jede Düse (22) ein erstes Aufnahmesegment (24) für das Biogas und ein zweites Segment (25) besitzt, das sich in Bezug auf das erste Segment (24) quer erstreckt, und eine Einspeisöffnung (23) für den Strahl definiert.

3. Reaktor gemäß Anspruch 2, **dadurch gekennzeichnet, dass** die Öffnung (23) auf einer geneigten Ebene bezüglich der Erstreckungsrichtung des zweiten Segments (25) der Düse (22) liegt

4. Reaktor gemäß Anspruch 2 oder 3, **dadurch gekennzeichnet, dass** sich das zweite Segment (25) der Düse (22) parallel zur Achse (A) erstreckt.

5. Reaktor gemäß einem der Ansprüche 2 bis 4, **dadurch gekennzeichnet, dass** sich das erste Segment (24) der Düse (22) senkrecht zur Achse (A) erstreckt.

6. Reaktor gemäß einem der vorherigen Ansprüche, **dadurch gekennzeichnet, dass** die Förderleitung (26) einen Hauptteil aufweist, der sich außerhalb der Kammer (6) erstreckt, und einen Endteil bildet, der durch die Seitenwand (8) verläuft und strömungsmittelmäßig mit dem ersten Segment (24) der Düse (22) verbunden ist.

7. Reaktor gemäß einem der vorherigen Ansprüche, **dadurch gekennzeichnet, dass** sich die Wand (8) um die Achse (A) herum erstreckt, und dass die Kammer (6) Folgendes aufweist:
eine obere Wand (9), welche quer ist und auf die Achse (A) trifft, die auf einer Seite der Seitenwand (8) angeordnet ist und eine erste Auslassöffnung (13) für das Biogas definiert, welches durch die Reaktion erzeugt wird; wobei die Welle (20), überhängend von der oberen Wand (9) innerhalb der Kammer (6) hervorragt; und
eine untere Wand (10), welche quer ist und auf die Achse (A) trifft, die auf einer mit Bezug auf die obere Wand (9) gegenüberliegenden Seite der Seitenwand (8) angeordnet ist und geeignet ist, um infolge der Schwerkraft mindestens einen Anteil des Restes zu sammeln; wobei die untere Wand (10) ferner eine zweite Auslassöffnung (15) für den Anteil des Restes definiert.

8. Reaktor gemäß Anspruch 7 und einem der Ansprüche 2 bis 6, **dadurch gekennzeichnet, dass** sich das zweite Segment (25) der Düse (22) von dem ersten Segment (24) zu der unteren Wand (10) hin erstreckt.

9. Reaktor gemäß einem der vorherigen Ansprüche, **dadurch gekennzeichnet, dass** er ein Kompressor- bzw. Verdichterglied (29) aufweist, welches das in der Kammer (6) produzierte Biogas aufnimmt, geeignet um das Biogas zu komprimieren, und strömungsmittelmäßig mit der Förderleitung (26) verbunden ist, um das Biogas, welches in die Kammer (6) geliefert werden soll, unter Druck zu setzen.

10. Anlage zur Produktion von Biogas aus nassem Abfall, welche Folgendes aufweist:
eine Vorbehandlungseinheit (2), welche nassen Abfall aufnimmt und die geeignet ist, um nassen Abfall zu zerkleinern und zu sieben;
einen Reaktor (4) gemäß einem der vorhergehenden Ansprüche, der strömungsmittelmäßig mit der Vorbehandlungseinheit (2) verbunden ist und der strömungsmittelmäßig mit einer Behandlungseinheit (14) für den Rest verbunden ist; und
einen Tank (12), der strömungsmittelmäßig mit dem Reaktor (4) verbunden ist und geeignet ist, das in der Kammer (6) produzierte Biogas zu speichern.

## Revendications

1. Réacteur (4) pour la production anaérobie de biogaz à partir de déchets humides prétraités, comprenant :
- une chambre de réaction (6) pouvant être alimentée avec un mélange comprenant lesdits déchets humides, et adaptée pour contenir une réaction biochimique dans un environnement anaérobie, dans laquelle ledit mélange est transformé en un biogaz et un rejet ; et
- des moyens d'agitation (20, 22) comprenant au moins un arbre (20) rotatif à l'intérieur de ladite chambre (6) autour d'un axe (A) et interagissant, en utilisation, avec ledit mélange pour maintenir celui-ci dans un état de mouvement continu dans ladite chambre (6) ;
lesdits moyens d'agitation (20, 22, 23, 26, 27) comprenant des moyens d'alimentation d'un jet de biogaz dans ladite chambre (6) ; lesdits moyens d'alimentation (22, 23, 26, 27) pouvant être alimentés avec ledit biogaz produit par ladite réaction biochimique et traversant une paroi latérale (8) de ladite chambre (6) faisant face audit arbre (20) ;
**caractérisé en ce que** lesdits moyens d'alimentation (22, 23, 26, 27) comprennent :
- deux ouvertures d'alimentation (23) dudit jet de biogaz à l'intérieur de ladite chambre (6) dans une position adjacente à ladite paroi latérale (8) pour maintenir au moins une région dudit mélange espacée dudit arbre (20) et adjacente à ladite paroi latérale (8) dans un état d'agitation continue ;
- deux buses (22) définissant des ouvertures d'alimentation (23) dudit jet de biogaz ;
- deux tuyaux de transport (26) dudit biogaz produit dans ladite chambre (6) et en raccordement fluidique avec lesdites buses (22) ;
- deux vannes d'ajustement (28), chacune étant intercalée le long d'un tuyau correspondant (26) ; et
- une unité de commande agissant, en utilisation, sur lesdites vannes (28) de manière à maintenir au moins une desdites vannes (28) ouvertes à la fois tandis que l'autre desdites vannes (28) reste fermée, et permettre que ledit biogaz s'écoule dans un seul tuyau (26) à la fois et permettre qu'une seule buse (22) correspondante à la fois alimente ledit jet de biogaz dans ladite chambre (6) ; le tuyau (26) le long duquel ledit flux peut varier au cours du temps, en utilisation, de manière à empêcher la solidification dudit mélange.

2. Réacteur selon la revendication 1, **caractérisé en ce que** chacune desdites buses (22) a un premier segment de réception (24) dudit biogaz et un deuxième segment (25) s'étendant transversalement par rapport au premier segment (24) et définissant ladite ouverture d'alimentation (23) dudit jet.

3. Réacteur selon la revendication 2, **caractérisé en ce que** ladite ouverture (23) est disposée sur un plan incliné par rapport à la direction d'extension dudit deuxième segment (25) de ladite buse (22) .

4. Réacteur selon la revendication 2 ou 3, **caractérisé en ce que** ledit deuxième segment (25) de ladite buse (22) s'étend parallèlement audit axe (A).

5. Réacteur selon l'une quelconque des revendications 2 à 4, **caractérisé en ce que** ledit premier segment (24) de ladite buse (22) s'étend de façon orthogonale par rapport audit axe (A).

6. Réacteur selon l'une quelconque des revendications précédentes, **caractérisé en ce que** ledit tuyau de transport (26) comprend une partie principale s'étendant à l'extérieur de ladite chambre (6) et présente une partie d'extrémité traversant ladite paroi latérale (8) et étant en raccordement fluidique avec ledit premier segment (24) de ladite buse (22).

7. Réacteur selon l'une quelconque des revendications précédentes, **caractérisé en ce que** ladite paroi latérale (8) s'étend autour de l'axe (A), et **en ce que** ladite chambre (6) comprend :
- une paroi supérieure (9) transversale et incidente par rapport audit axe (A), agencée sur un côté de ladite paroi latérale (8), et définissant une première ouverture de sortie (13) pour ledit biogaz généré par ladite réaction ; ledit arbre (20) faisant saillie au-dessus de ladite paroi supérieure (9) à l'intérieur de ladite chambre (6) ; et
- une paroi inférieure (10) transversale et incidente par rapport audit axe (A), agencée sur le côté opposé de ladite paroi latérale (8) par rapport à ladite paroi supérieure (9), et adaptée pour collecter, par gravité, au moins une fraction dudit rejet ; ladite paroi inférieure (10) définissant une deuxième ouverture de sortie (15) pour ladite fraction dudit rejet.

8. Réacteur selon la revendication 7, dépendant de l'une quelconque des revendications 2 à 6, **caractérisé en ce que** ledit deuxième segment (25) de ladite buse (22) s'étend dudit premier segment (24) vers ladite paroi inférieure (10).

9. Réacteur selon l'une quelconque des revendications précédentes, **caractérisé en ce qu'**il comprend un élément de compresseur (29) recevant ledit biogaz produit dans ladite chambre (6), adapté pour comprimer ledit biogaz et en raccordement fluidique avec ledit tuyau de transport (26) de manière à mettre sous pression ledit biogaz destiné à être alimenté dans ladite chambre (6).

10. Installation pour la production de biogaz à partir de déchets humides, comprenant :
- une unité de prétraitement (2) recevant lesdits déchets humides et adaptée pour broyer et cribler lesdits déchets humides ;
- un réacteur (4) selon l'une quelconque des revendications précédentes en raccordement fluidique avec ladite unité de prétraitement (2) et en raccordement fluidique avec une unité de traitement (14) dudit rejet ; et
- un réservoir (12) en raccordement fluidique avec ledit réacteur (4) et adapté pour stocker ledit biogaz produit dans ladite chambre (6).
